# EUROPEAN PATENT APPLICATION

(11) **EP 3 357 659 A1**
(43) Date of publication of application: **08.08.2018**
(21) Application number: 16851069.1
(22) Date of filing: 07.09.2016
(51) Int. Cl.: B29C 33/40, B29C 39/02, B81C 99/00

(54) **PATTERNED SHEET PRODUCTION METHOD**

(30) Priority: 30.09.2015 JP 2015193481
(71) Applicant: FUJIFILM Corporation, Tokyo 106-8620 (JP)
(72) Inventor: OGAWA, Shotaro, Ashigarakami-gun Kanagawa 258-8577 (JP); USA, Toshihiro, Ashigarakami-gun Kanagawa 258-8577 (JP); FUJITA, Atsushi, Ashigarakami-gun Kanagawa 258-8577 (JP)
(74) Representative: Klunker IP Patentanwälte PartG mbB
(86) International application number: PCT/JP2016/076341
(87) International publication number: WO 2017/056895

(57) **Abstract**

Provided is a manufacturing method of a pattern sheet having excellent productivity at low costs. The manufacturing method of a pattern sheet includes: a process of producing, using a plate precursor 10 having a protruding pattern 12 formed thereon, a plurality of first molds 20 having a recessed shape, which is an inverted shape of the protruding pattern, from a resin; a process of producing a plurality of metallic electroforms 70 having the protruding pattern formed thereon by electroforming using the first mold 20; a process of producing a plurality of second molds 80 having a recessed shape, which is an inverted shape of the protruding pattern, from a resin using the electroform 70; and a process of producing pattern sheets 100 and 110 having a protruding pattern 102 formed thereon using the second mold 80.

## Description

### BACKGROUND OF THE INVENTION

### 1. Field of the Invention

The present invention relates to a manufacturing method of a pattern sheet, and particularly to a manufacturing method of a protruding pattern sheet in which a large number of pattern sheets are manufactured based on a plate precursor having a protruding pattern formed therein.

### 2. Description of the Related Art

In the related art, as a method of administering a medicine or the like through a living body surface, that is, the skin or mucous membrane, a method of attaching a liquid substance or a powdery substance has been mostly used. However, since a region to which such substances are attached is limited to the surface of the skin, there may be cases where a medicine or the like attached thereto are removed by perspiration or contact with foreign matter, and it is difficult to administer an appropriate amount of the medicine. In addition, in order to cause the medicine to penetrate deep into the skin, it is difficult to reliably control the penetration depth in such a method utilizing penetration by diffusion of the medicine. Therefore, it is difficult to obtain a sufficient medicinal effect.

Therefore, a method of injecting a medicine by inserting a small needle into the skin using a percutaneous absorption sheet on which a needle-like protrusion (also referred to as small needle or microneedle) which contains a drug and has a high aspect ratio is formed is employed.

As a method of manufacturing a functional film having such a high aspect ratio structure, for example, in JP2008-245955A, JP2011-194594A, and JP2014-519344, it is described that a sheet is manufactured by molding a resin or by electroforming using a mold having a protruding or recessed pattern.

### SUMMARY OF THE INVENTION

However, the method of manufacturing a functional film having a high aspect ratio structure described in JP2008-245955A, JP2011-194594A, and JP2014-519344 is not suitable for mass production. In a case of manufacturing a large number of fine pattern formed products using a plate precursor on which a fine pattern having an aspect ratio structure is formed, costs and productivity become important. First, the processing cost of the plate precursor becomes extremely high as the pattern shape becomes finer and more complex. Furthermore, since durability is also limited, it is preferable to produce a large number of duplicates of the plate precursor. Although formed products are produced using the duplicated product as a mold, there are limits or problems that (1) there is a need to produce an accurate duplicated product at low costs, (2) there is a limit to the durability of the duplicated product, (3) in a case where the fine pattern has a high aspect ratio shape, it becomes difficult to peel the fine pattern, (4) stable production without defects is preferable, and (5) there is a case where a mold for forming a final product is for a single use (disposable). The techniques described in JP2008-245955A, JP2011-194594A, and JP2014-519344 are not suitable for the process in which mass production is assumed, and particularly a technique for manufacturing a large number of molds at low costs is desirable.

In a case of molding a functional film having a high aspect ratio structure, when the functional film is peeled away from the mold, the mold may be damaged, and in this case, it is not possible to reuse the mold. In a case where the functional film is a medicine, high costs are needed to clean the mold. In this case, the mold is disposable, and a large number of molds are necessary.

The present invention has been made taking the above circumstances into consideration, and an object thereof is to provide a manufacturing method of a pattern sheet in which low costs are needed and productivity is improved.

In order to achieve the object, the present invention provides a manufacturing method of a pattern sheet comprising: a process of producing, using a plate precursor having a protruding pattern formed therein, a plurality of first molds having a recessed shape, which is an inverted shape of the protruding pattern, from a resin; a process of producing a plurality of metallic electroforms having the protruding pattern formed therein by electroforming using the first mold; a process of producing a plurality of second molds having a recessed shape, which is an inverted shape of the protruding pattern, from a resin using the electroform; and a process of manufacturing a pattern sheet having the protruding pattern formed therein using the second mold.

According to the present invention, the plurality of first molds are produced using the plate precursor, the plurality of electroforms are produced using the first mold, and the plurality of second molds are produced using the electroform. Therefore, a large number of the second molds having the inverted shape of the protruding pattern formed on the plate precursor recessed shape can be produced using the single plate precursor. Accordingly, using the second mold, a large number of pattern sheets can be manufactured at low costs.

In another aspect of the present invention, it is preferable that the manufacturing method of a pattern sheet further comprises: after the process of producing the plurality of first molds, a process of producing a first assembled mold by joining the plurality of first molds to increase an area of surfaces of the first molds on which the recessed shapes are formed.

According to this aspect, after the first assembled mold which is increased in area by joining the plurality of first molds is produced, the electroforms are produced by electroforming, and thus the plurality of electroforms having the protruding pattern formed therein can be produced by a single electroforming process, thereby improving productivity.

In another aspect of the present invention, it is preferable that the plate precursor is processed by machining.

According to this aspect, by processing the plate precursor through machining, a desired shape can be produced with good accuracy.

In another aspect of the present invention, it is preferable that the resin forming the second mold is a silicone resin or a thermoplastic resin.

According to this aspect, since a silicone resin or a thermoplastic resin is used as the resin forming the second mold, even in a case where the pattern sheet is a medicine, the pattern sheet maintaining safety can be manufactured.

In another aspect of the present invention, it is preferable that a material of the pattern sheet is a water-soluble material.

According to this aspect, by using the water-soluble material as the material of the pattern sheet, in a case where the protruding pattern formed on the pattern sheet is inserted into the skin, injection of a medicine can be facilitated.

In another aspect of the present invention, it is preferable that the resin forming the first mold is an ultraviolet curable resin or a thermoplastic resin.

According to this aspect, by using the ultraviolet curable resin or thermoplastic resin is used as the resin forming the first mold, the first mold can be easily formed at low costs.

In another aspect of the present invention, it is preferable that a through-hole is formed at a distal end of the recessed shape in the second mold.

According to this aspect, since the through-hole is provided at the distal end of the recessed shape of the second mold, in a case where the pattern sheet is manufactured, the injection of the material into the recessed shape can be easily performed.

In another aspect of the present invention, it is preferable that the manufacturing method of a pattern sheet further comprises: after the process of producing the plurality of second molds, a process of producing a second assembled mold by joining the plurality of second molds to increase an area of surfaces of the second molds on which the recessed shapes are formed.

According to this aspect, by manufacturing the pattern sheet after producing the second assembled mold which is increased in area by joining the plurality of second molds, the pattern sheet having a plurality of the protruding patterns formed therein can be manufactured in a single pattern sheet manufacturing process, thereby improving productivity.

According to the manufacturing method of a pattern sheet of the present invention, the pattern sheet having the protruding pattern can be manufactured at low costs, and productivity can be improved.

### BRIEF DESCRIPTION OF THE DRAWINGS

Figs. 1(A) to 1(E) are process diagrams illustrating a procedure for producing an electroform.
Figs. 2(A) to 2(D) are process diagrams illustrating a procedure for manufacturing a pattern sheet.
Figs. 3(A) to 3(F) are process diagrams illustrating a procedure for producing a first assembled mold.
Fig. 4 is a plan view of the first assembled mold.

### DESCRIPTION OF THE PREFERRED EMBODIMENTS

Hereinafter, a manufacturing method of a pattern sheet according to the present invention will be described with reference to the accompanying drawings. In this specification, "to" is used to include numerical values described before and after "to" as the lower limit and the upper limit.

The present invention is a technique for manufacturing, using a plate precursor having a protruding pattern formed therein, a pattern sheet having a protruding pattern formed therein as a duplicate of the plate precursor. Specifically, using a plate precursor 10 having a protruding pattern 12 formed therein, a plurality of first molds 20 having a recessed shape, which is an inverted shape of a protruding pattern 12, are produced. Next, a plurality of metallic electroforms 70 are produced by electroforming using the first mold 20. In addition, using the electroform 70, a plurality of second molds 80 having a recessed shape, which is an inverted shape of a protruding pattern, are produced. Last, a pattern sheet 100 having a protruding pattern 102 formed therein is manufactured using the second mold 80. As described above, using the plate precursor 10, a plurality of the first molds 20, a plurality of the electroforms 70, and a plurality of the second molds 80 are produced, so that a large number of the second molds 80 can be produced. Therefore, a pattern sheet 110 is manufactured using the second mold 80, so that the pattern sheet 110 as a duplicate of the plate precursor 10 can be manufactured. Hereinafter, each process will be described.

### <Manufacturing Method of Pattern Sheet>

[Process of Producing First Mold] A process of producing the first mold 20 is a process of producing, using the plate precursor 10 having the protruding pattern 12 formed therein, a plurality of the first molds 20 having a recessed shape 22, which is an inverted shape of the protruding pattern 12, from a resin.

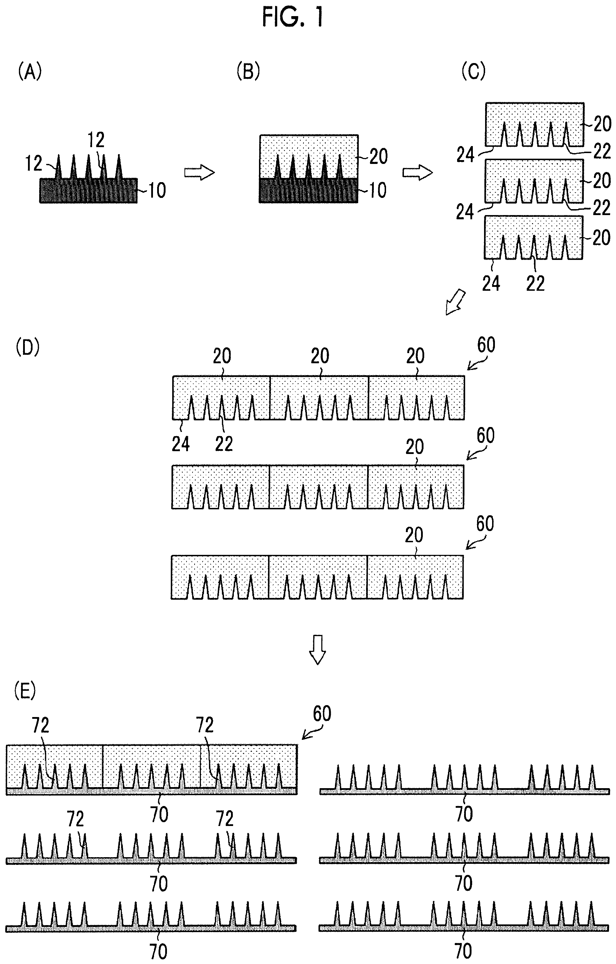

First, as illustrated in (A) of Fig. 1, the plate precursor 10 having the protruding pattern 12 formed therein is prepared. Although a method of producing the plate precursor 10 having the protruding pattern 12 formed therein is not particularly limited, for example, the plate precursor 10 having the protruding pattern 12 formed therein can be produced as follows. A plurality of the protruding patterns 12 can be produced on the surface of the plate precursor 10 by processing a metal substrate made of Ni or the like through machining using a cutting tool such as a diamond insert bite.

As another method, a photoresist is applied onto a Si substrate, and exposure and development are performed thereon. In addition, etching such as reactive ion etching (RIE) is performed, thereby producing the protruding patterns 12 on the surface of the plate precursor 10. Furthermore, in the case where etching such as RIE is performed to form the protruding patterns 12 on the surface of the plate precursor 10, it is possible to form the protruding patterns by performing etching in an oblique direction while rotating the Si substrate.

In this embodiment, it is preferable that the plate precursor 10 is processed by machining. A desired shape can be accurately produced by machining.

Next, as illustrated in (B) and (C) of Fig. 1, a plurality of the first molds 20 having a recessed shape 22 are produced using the plate precursor 10. As a method of producing the first molds 20, the production can be performed using a resin by the following methods. In the first method, a silicone resin obtained by adding a hardener to PDMS (polydimethylsiloxane, for example, SYLGARD 184 manufactured by Dow Corning Corporation, SYLGARD: trademark) is poured to the plate precursor 10 and is subjected to a heat treatment at 100°C to cure, and thereafter, a first mold having a recessed shape, which is an inverted shape of a protruding pattern is produced using the plate precursor.

The second method is a method in which an ultraviolet curable resin which is cured by being irradiated with ultraviolet radiation is poured to the plate precursor 10 and is irradiated with ultraviolet radiation in a nitrogen atmosphere, and thereafter the first mold 20 is peeled away from the plate precursor 10. The third method is a method in which a solution obtained by dissolving a plastic resin such as polystyrene or PMMA (polymethyl methacrylate) in an organic solvent is poured to the plate precursor 10 to which a release agent is applied and is dried so as to cause the organic solvent to be volatilized for curing, and thereafter the first mold 20 is peeled away from the plate precursor 10.

The material forming the first mold 20 is a resin, and an ultraviolet curable resin or a thermoplastic resin is preferably used. By using an ultraviolet curable resin or a thermoplastic resin, the production of the first mold 20 can be facilitated, and the protruding pattern 12 of the plate precursor 10 can be stably formed.

A plurality of the first molds 20 are produced using the plate precursor 10. The number of first molds produced using a single plate precursor is preferably ten or more, more preferably 100 or more, and even more preferably 500 or more.

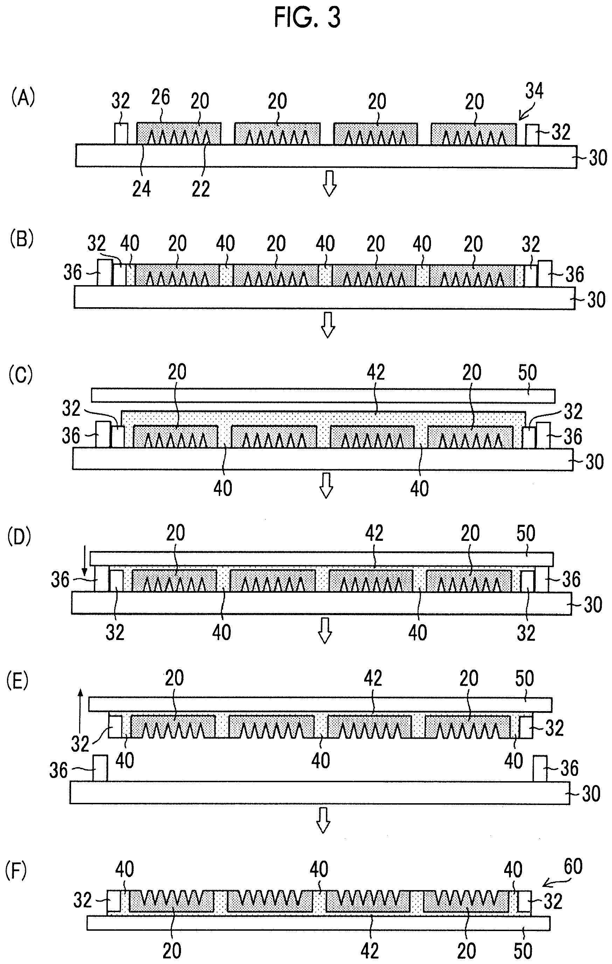

[Process of Producing First Assembled Mold] Next, as illustrated in (D) of Fig. 1, by joining a plurality of the first molds 20 produced using the plate precursor 10, the surfaces of the first molds 20 on which the recessed shapes 22 are formed are increased in area, such that a first assembled mold 60 is produced. Figs. 3(A) to 3(F) are process diagrams of the production of the first assembled mold 60.

First, as illustrated in (A) of Fig. 3, the plurality of the first molds 20 are disposed on a first substrate 30 so as to be spaced apart from each other. A frame 32 having an opening 34 is provided on the first substrate 30. In the region of the opening 34 of the frame 32, the first substrate 30 is exposed. In addition, the plurality of the first molds 20 are disposed in the region of the opening 34 of the frame 32, that is, inside the frame 32.

The plurality of the first molds 20 are disposed on the first substrate 30 so as to be spaced apart from each other and are disposed at positions where surfaces 24 on which the recessed shapes 22 are formed are in contact with the first substrate 30. The flatness of the surface 24 on which the recessed shape of each of the first molds 20 is formed is secured by the first substrate 30. That is, in a state where the surfaces 24 on which the recessed shapes 22 are formed are flush with each other, the position of each of the first molds 20 is defined by the first substrate 30. As the first substrate 30, a quartz glass plate or the like can be used. In particular, although the material of the first substrate 30 is not limited, a material having high stiffness is preferable. This is because the position of each of the first molds 20 is defined by the first substrate 30.

As the material of the frame 32, acrylic, PP (polypropylene), PC (polycarbonate), or the like can be used. However, the frame 32 may not be provided. For example, in a case where an adhesive 40 has high viscosity and low fluidity, the frame 32 may not be provided.

In addition, it is preferable that the plurality of the first molds 20 are disposed on the first substrate 30 under an environment at reduced pressure. By disposing the first molds 20 on the first substrate 30 under an environment at reduced pressure, air in the recessed shapes 22 of the first molds 20 can be reduced.

In a case of filling an adhesive, it is preferable that the first molds 20 and the first substrate 30 are not misaligned from each other, and in a case where the first molds 20 are peeled away from the first substrate 30, it is preferable that the first molds 20 are easily peeled away from the first substrate 30. Therefore, in the case of disposing the first molds 20 on the first substrate 30, it is preferable that the first molds 20 and the first substrate 30 are brought into contact with a thermal release sheet or ultraviolet release sheet interposed therebetween. The thermal release sheet refers to an adhesive sheet having a property that the adhesive force is decreased by heating the thermal release sheet to a predetermined temperature or higher. The ultraviolet release sheet refers to an adhesive sheet having a property that the adhesive force is decreased by irradiating the ultraviolet release sheet with ultraviolet radiation.

The first molds 20 and the first substrate 30 may be brought into direct contact with each other, or may be brought into indirect contact with a release sheet such as the thermal release sheet or ultraviolet release sheet interposed therebetween.

Next, as illustrated in (B) and (C) of Fig. 3, the adhesive 40 is supplied to the periphery of each of the first molds 20 on the first substrate 30. First, the adhesive 40 is supplied between the first molds 20 on the first substrate 30. In the case where the frame 32 is provided on the first substrate 30, the adhesive 40 is supplied between the first molds 20 and the frame 32. Next, the adhesive 40 is cured. It is preferable to use an ultraviolet curable adhesive as the adhesive 40. In a case where the adhesive 40 is an ultraviolet curable adhesive, the adhesive 40 is cured by irradiating the adhesive 40 with ultraviolet radiation. The adhesive 40 is not limited to the ultraviolet curable adhesive, and other adhesives can also be used.

Next, as illustrated in (C) of Fig. 3, an adhesive 42 is supplied to surfaces 26 of the plurality of the first molds 20 on the side opposite to the surfaces 24 on which the recessed shapes 22 are formed. It is preferable to use an ultraviolet curable adhesive as the adhesive 42, and it is preferable that the adhesive 42 has the same composition as the adhesive 40. The adhesive 42 is not limited to the ultraviolet curable adhesive, and other adhesives can also be used.

As described above, regarding the supply of the adhesive, the adhesive is supplied in two stages in which the adhesive 40 is initially supplied and cured between the first molds 20 and the adhesive 42 is thereafter supplied to the surfaces 26 of the first molds 20 on the side opposite to the surfaces 24 on which the recessed shapes 22 are formed, thereby suppressing warping, deformation, and the like.

In addition, since the surfaces 24 of the first molds 20 on which the recessed shapes 22 are formed and the adhesive 40 are supported by the first substrate 30, the surfaces 24 of the first molds 20 on which the recessed shapes 22 are formed and the adhesive 40 are flush with each other. Therefore, occurrence of steps, deformation, or the like between the first molds 20 and the adhesive 40 can be suppressed.

After supplying the adhesive 42, as illustrated in (D) of Fig. 3, a second substrate 50 is bonded to the surfaces 26 of the plurality of the first molds 20 on the side opposite to the surfaces 24 on which the recessed shapes 22 are formed.

After supplying the adhesive 42, the second substrate 50 is pressed against the adhesive 42. Thereafter, the adhesive 42 is cured such that the surfaces 26 of the first molds 20 on the side opposite to the surfaces 24 on which the recessed shapes 22 are formed and the second substrate 50 are bonded with the adhesive 42 interposed therebetween.

In this embodiment, a spacer 36 is provided around the frame 32. In the case where the second substrate 50 is pressed against the adhesive 42, the second substrate 50 is brought into contact with the spacer 36. The distance between the first substrate 30 and the second substrate 50 can be defined by the spacer 36.

Tthe spacer 36 may or may not be provided. In a case where the spacer 36 is not provided, the distance between the first substrate 30 and the second substrate 50 can be defined by bringing the second substrate 50 into contact with the frame 32.

By setting the height of the spacer 36 to be higher than the height of the frame 32, in the case where the second substrate 50 is pressed against the adhesive 42, the adhesive 42 can be incorporated between the frame 32 and the second substrate 50.

In the case where the adhesive 42 is the ultraviolet curable adhesive, the adhesive 42 can be cured by irradiating the adhesive 42 with ultraviolet radiation. In the case of irradiating the adhesive 42 with ultraviolet radiation, it is preferable that the second substrate 50 is a material capable of transmitting ultraviolet radiation.

Next, as illustrated in (E) of Fig. 3, the first molds 20 and the first substrate 30 are peeled away from each other. The first molds 20 and the first substrate 30 may be peeled away from each other by moving the first molds 20 in a direction away from the first substrate 30 or by separating the first substrate 30 from the first molds 20. Alternatively, the first molds 20 and the first substrate 30 may be moved in directions away from each other.

In a case where the first molds 20 and the first substrate 30 are adhered with the thermal release sheet or ultraviolet ray release sheet interposed therebetween, the adhesive force between the first molds 20 and the thermal release sheet or the adhesive force between the first molds 20 and the ultraviolet release sheet is decreased by heating the thermal release sheet to a predetermined temperature or higher or irradiating the ultraviolet release sheet with ultraviolet radiation. Accordingly, the first molds 20 and the first substrate 30 can be easily peeled away from each other. In addition, it is preferable that the thermal release sheet or the ultraviolet release sheet is peeled away from the plurality of the first molds 20 together with the first substrate 30.

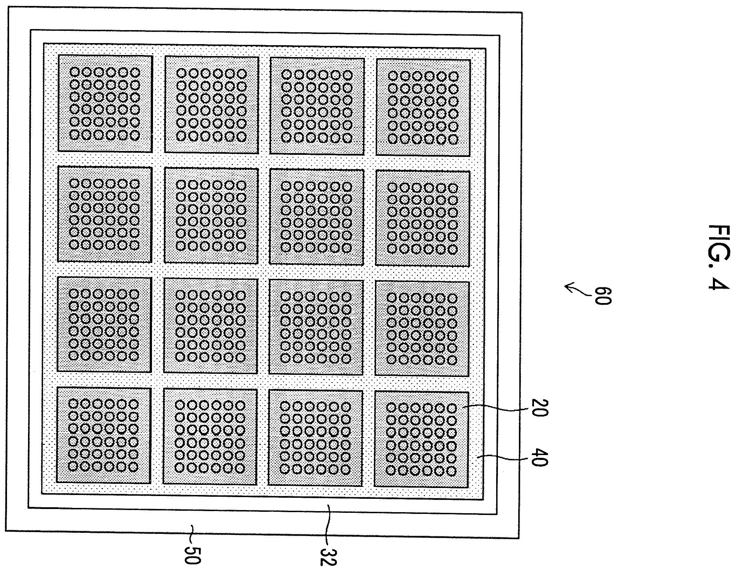

(F) of Fig. 3 is a sectional view of the first assembled mold 60 formed by the above-described method. By peeling the first molds 20 and the first substrate 30 away from each other, the first assembled mold 60 is completed. As illustrated in (F) of Fig. 3 and 4, the first assembled mold 60 has a plurality of the first molds 20. Fig. 4 illustrates the first assembled mold 60 having 4 × 4 = 16 first molds 20. The plurality of the first molds 20 are disposed on one surface of the second substrate 50 and are integrated by the adhesives 40 and 42.

In (D) of Fig. 1, the first assembled mold 60 is illustrated in a simplified manner. However, the actual structure thereof is as illustrated in Figs. 3(A) to 3(F).

The production of the first assembled mold 60 may or may not be performed. By producing the electroform 70 in the subsequent process using the first assembled mold 60, the electroform 70 having a large area can be produced by a single electroforming process. Furthermore, in the subsequent processes, the pattern sheet 100 is manufactured using the electroform 70 having a large area, and the second mold 80 or a second assembled mold 90, thereby improving productivity. For example, in a method of producing an electroform directly using an initial plate precursor, when the electroformed product is peeled away from the plate precursor, the plate precursor (particularly a needle portion) is easily damaged, and thus great costs are incurred by fatal defects, or breaking or replacement of the plate precursor, which is not preferable.

[Process of Producing Electroform] Returning to Fig. 1, (E) of Fig. 1 is a diagram illustrating a process of producing the metallic electroform 70 having protruding patterns 72 formed therein by electroforming using the first assembled mold 60. In (E) of Fig. 1, the first assembled mold 60 is used. However, as described above, the electroform may also be produced using the first mold 20.

In the electroforming process, the first assembled mold 60 is disposed at a cathode body, and an electroformed matter is formed on the surface of the first assembled mold 60 such that the metallic electroform 70 which is paired with the first assembled mold 60 is produced.

Also in the production of the electroform 70 by the electroforming process, a plurality of the electroforms 70 are produced using the single first mold 20 or the first assembled mold 60. By producing the plurality of the electroforms 70 using the first mold 20 or the first assembled mold 60, the plurality of the electroforms 70 can be produced using the single plate precursor 10. Regarding the production of the electroforms 70, the number of the electroforms 70 produced using the first mold 20 or the single first assembled mold 60 is preferably one or more, more preferably three or more, and even more preferably five or more.

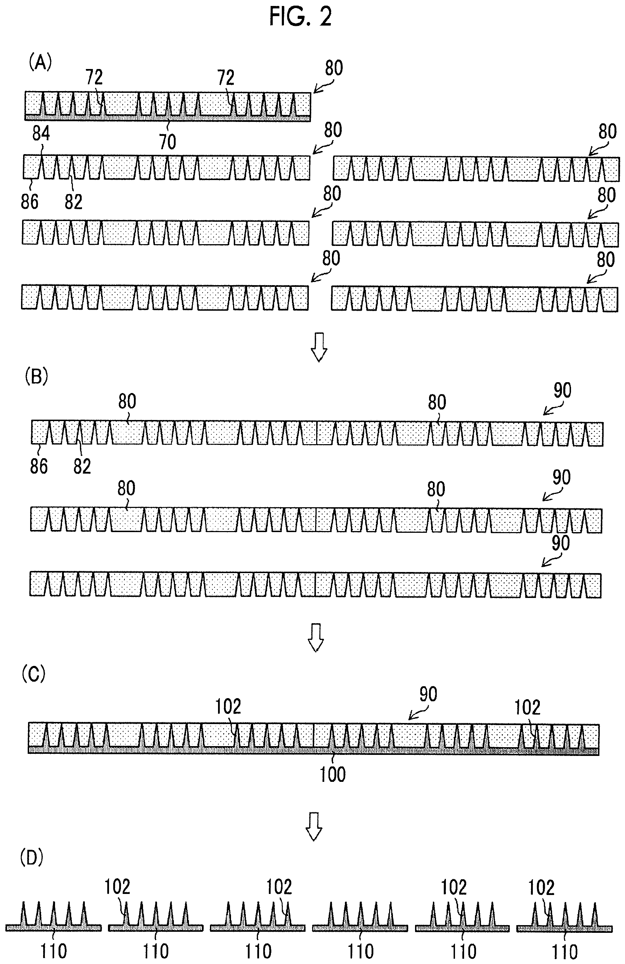

[Process of Producing Second Mold] After producing the metallic electroform 70 by the electroforming process, as illustrated in (A) of Fig. 2, the second mold 80 with a recessed shape 82 is produced from a resin using the electroform 70.

The second mold 80 can be produced by a method similar to the method of producing the first mold 20. The resin forming the second mold 80 is preferably a silicone resin or a thermoplastic resin. By using the silicone resin or thermoplastic resin as the resin forming the second mold 80, safety can be secured in a case where the pattern sheets 100 and 110 manufactured using the second mold 80 are used for a living body or the like.

In the production of the second mold, it is preferable that the thickness of the second mold 80 after the formation is smaller than the height of the protruding pattern 72 of the electroform 70. By causing the thickness of the second mold 80 to be smaller than the height of the protruding pattern 72 of the electroform 70, through-holes 84 can be formed at the distal ends of the recessed shapes 82 of the second mold 80, which is an inverted shape of the protruding pattern 72 of the electroform 70. However, in a case where the thickness of the second mold 80 is too small compared to the height of the protruding pattern 72 of the electroforming 70, the shape of the distal end of the protruding patterns 102 of the manufactured pattern sheets 100 and 110 is rounded, and it becomes difficult for the distal end thereof to be inserted into the skin, which is not preferable. The thickness of the second mold 80 is preferably set to a height lower than the height of the protruding pattern 72 of the electroform 70 by 10 to 50 µm. By forming the through-holes 84 in the second mold 80, in a subsequent process of manufacturing the pattern sheet 100, the material of the pattern sheet 100 can be easily injected into the recessed shapes 82 of the second mold 80. In some cases, a mold without through-holes may be produced.

A plurality of the second molds 80 are produced using the electroform 70 formed by electroforming the number of the second molds 80 produced using the single electroform 70 is preferably 100 or more, more preferably 300 or more, and even more preferably 500 or more.

[Process of Producing Second Assembled Mold] Next, as illustrated in (B) of Fig. 2, by joining the second molds 80, surfaces 86 of the second molds 80 on which the recessed shapes 82 are formed are increased in area, such that the second assembled mold 90 is produced. As a method of producing the second assembled mold 90, the second assembled mold 90 can be produced by the same method as in the process of producing the first assembled mold 60.

The production of the second assembled mold 90 may or may not be performed. By producing the pattern sheet 110 as in the subsequent process using the second assembled mold 90, the pattern sheet 110 is manufactured once, and a plurality of pattern sheets corresponding to the plate precursor 10 can be manufactured, thereby improving productivity.

[Process of Manufacturing Pattern Sheet] (C) of Fig. 2 is a diagram illustrating a process of manufacturing the pattern sheet 100 using the second assembled mold 90. Although the second assembled mold 90 is used in (C) of Fig. 2, the pattern sheet may also be formed using the second mold 80.

There may be cases where the second mold 80 or the second assembled mold 90 is used only once and is preferably disposable. In a case where the pattern sheet 100 is used as a medicine, the second mold 80 or the second assembled mold 90 is preferably disposable in consideration of the safety of the manufactured pattern sheet 100 for a living body. In addition, by causing the second mold 80 or the second assembled mold 90 to be disposable, there is no need to clean the second mold 80 and the second assembled mold 90, thereby reducing cleaning costs. In particular, in the case where the pattern sheet 100 is used as a medicine, high cleaning performance is required, and the cleaning costs are high.

It is preferable that a water-soluble material is used as the material forming the pattern sheet 100. As a material of a resin polymer of a polymer solution used for the manufacturing of a pattern sheet, it is preferable to use a biocompatible resin. As such resins, sugars such as glucose, maltose, pullulan, sodium chondroitin sulfate, sodium hyaluronate, and hydroxyethyl starch, proteins such as gelatin, and biodegradable polymers such as polylactic acid and a lactic acid-glycolic acid copolymer are preferably used. Among these, since gelatin-based materials have adhesiveness to many base materials and have a strong gel strength in a case of being used as a gelating material, the gelatin-based materials can be brought into close contact with a base material, and the pattern sheet 100 can be peeled away using the base material (not illustrated) in a case where the pattern sheet 100 is peeled away from the second mold 80 or the second assembled mold 90. Therefore, the gelatin-based materials can be suitably used. Although the concentration varies depending on the material, it is preferable that the concentration is set so that 10 to 50 mass% of the resin polymer is contained in the polymer solution which does not contain the drug. The solvent used for dissolution may not be warm water as long as the solvent is volatile, and methyl ethyl ketone (MEK), alcohol, or the like may be used. In addition, it is possible to dissolve the drug, which is supplied into the body according to the application, in the solution of the polymer resin. The polymer concentration of the polymer solution containing the drug (the concentration of the polymer excluding the drug in a case where the drug itself is a polymer) is preferably 0 to 30 mass%.

As a method of preparing the polymer solution, in a case where a water-soluble polymer (such as gelatin) is used, a water-soluble powder may be dissolved in water and the drug may be added after the dissolution. Otherwise, a powder of a water-soluble polymer may be dissolved in a liquid in which the drug is dissolved. In a case where it is difficult to dissolve the polymer in water, heating may be performed for dissolution. The temperature can be appropriately selected depending on the kind of the polymer material, and it is preferable that heating is performed at a temperature of about 60°C or lower. For the solution containing the drug, the viscosity of the solution of the polymer resin is preferably 100 Pa·s or less, and more preferably 10 Pa·s or less. For a solution which does not contain a drug, the viscosity is preferably 2000 Pa·s or less, and more preferably 1000 Pa·s or less. By appropriately adjusting the viscosity of the solution of the polymer resin, injecting the solution into a needle-like recess of a mold is facilitated. For example, the viscosity of the solution of the polymer resin can be measured with a capillary viscometer, a falling ball viscometer, a rotational viscometer, or a vibrational viscometer.

The drug to be contained in the polymer solution is not limited as long as the drug has a function as a drug. Particularly, the drug is preferably selected from peptides, proteins, nucleic acids, polysaccharides, vaccines, pharmaceutical compounds belonging to water-soluble low molecular compounds, or cosmetic ingredients.

As a method of injecting the solution of the resin polymer into the second mold 80 or the second assembled mold, for example, application using a spin coater may be employed. In a case where the second assembled mold 90 is used for the manufacturing, it is conceivable that regarding the injection of the solution, the solution is added dropwise by a dispenser or the like only to the region where the recessed shapes 82 of the second assembled mold 90 are formed.

In a case where the through-holes 84 are provided at the distal ends of the recessed shapes 82 of the second mold 80, air in the recessed shapes 82 can be released through the through-holes 84 when the solution is injected into the recessed shapes 82. Therefore, the solution can be easily incorporated into the recessed shapes 82 of the second mold 80 or the second assembled mold 90. This process is desirably performed in a reduced pressure state.

After manufacturing the pattern sheet 100 which is the inverted shape of the second assembled mold 90, as illustrated in (D) of Fig. 2, the pattern sheets 110 having the protruding pattern 102 formed therein as a duplicate of the plate precursor 10 can be manufactured by cutting the pattern sheet 100.

The shape of the protrusion of the manufactured pattern sheet 110 is not particularly limited as long as the distal end thereof has a tapered shape, and examples of the shape include a conical shape, a triangular pyramid shape, and a square pyramid shape. The shape can also be formed of a needle portion having a tapered shape and a frustum portion connected to the needle portion.

In a case where the pattern sheet 110 is a microneedle, the drug is preferably contained in the protruding pattern 102. The protruding pattern 102 may be formed of a drug layer containing a drug and a polymer layer that does not contain a predetermined amount of the drug. In a case where the drug is mixed in the pattern sheet, by forming the drug layer at the distal end of the protruding pattern 102, the drug can be efficiently delivered into the skin. The polymer layer is formed in a portion of the protruding pattern 102 excluding the drug layer. For example, the needle portion may be formed of the drug layer, and the frustum portion may be formed of the polymer layer. Alternatively, distribution of the drug layer and the polymer layer in the needle portion and the frustum portion may be appropriately set.

The height of the protrusion of the protruding pattern is in a range of 100 µm to 2000 µm, and preferably in a range of 200 µm to 1500 µm.

Since the protruding pattern 102 of the manufactured pattern sheet 110 is a duplicate of the protruding pattern 12 of the plate precursor 10, the protruding pattern 102 of the pattern sheet 110 can have a desired shape by causing the shape of the protruding pattern 12 of the plate precursor 10 to have a desired shape.

According to the pattern sheet 110 manufactured by the above-described method, a plurality of the second molds 80 or the second assembled molds 90 are produced using the single plate precursor 10, and the pattern sheets 110 are manufactured using the second molds 80 or the second assembled molds 90. Accordingly, the costs of the plate precursor 10 can be reduced, and the manufacturing costs of the pattern sheets 110 can be reduced. In addition, by manufacturing a large number of the pattern sheets 110 using the single plate precursor 10, the pattern sheets 110 can be manufactured with good reproducibility.

### Explanation of References

10: plate precursor
12, 72, 82, 102: protruding pattern
20: first mold
22: recessed shape
24, 86: surface on which recessed shape is formed
26: surface on opposite side
30: first substrate
32: frame
34: opening
36: spacer
40, 42: adhesive
50: second substrate
60: first assembled mold
70: electroform
80: second mold
84: through-hole
90: second assembled mold
100, 110: pattern sheet

## Claims

1. A manufacturing method of a pattern sheet comprising:
a process of producing, using a plate precursor having a protruding pattern formed therein, a plurality of first molds having a recessed shape, which is an inverted shape of the protruding pattern, from a resin;
a process of producing a plurality of metallic electroforms having the protruding pattern formed therein by electroforming using the first mold;
a process of producing a plurality of second molds having a recessed shape, which is an inverted shape of the protruding pattern, from a resin using the electroform; and
a process of manufacturing a pattern sheet having the protruding pattern formed therein using the second mold.

2. The manufacturing method of a pattern sheet according to claim 1, further comprising
after the process of producing the plurality of first molds, a process of producing a first assembled mold by joining the plurality of first molds to increase an area of surfaces of the first molds on which the recessed shapes are formed.

3. The manufacturing method of a pattern sheet according to claim 1 or 2,
wherein the plate precursor is processed by machining.

4. The manufacturing method of a pattern sheet according to any one of claims 1 to 3,
wherein the resin forming the second mold is a silicone resin or a thermoplastic resin.

5. The manufacturing method of a pattern sheet according to any one of claims 1 to 4,
wherein a material of the pattern sheet is a water-soluble material.

6. The manufacturing method of a pattern sheet according to any one of claims 1 to 5,
wherein the resin forming the first mold is an ultraviolet curable resin or a thermoplastic resin.

7. The manufacturing method of a pattern sheet according to any one of claims 1 to 6,
wherein the second mold is provided with a through-hole at a distal end of the recessed shape.

8. The manufacturing method of a pattern sheet according to any one of claims 1 to 7, further comprising
after the process of producing the plurality of second molds, a process of producing a second assembled mold by joining the plurality of second molds to increase an area of surfaces of the second molds on which the recessed shapes are formed.
